# NEUE EUROPÄISCHE PATENTSCHRIFT

(11) **EP 0 447 585 B2**
(45) Veröffentlichungstag und Bekanntmachung des Hinweises auf die Entscheidung über den Einspruch: **07.05.2003**
(45) Hinweis auf die Patenterteilung: 03.05.1995
(21) Anmeldenummer: 90105405.6
(22) Anmeldetag: 22.03.1990
(51) Int. Cl.: C07K 1/02, C07K 1/16, A61K 39/395

(54) **Verfahren zur Herstellung eines intravenös verträglichen Immunglobulin-G-Präparates**
Process for manufacturing an intravenously tolerant immunoglobulin-G preparation
Procédé de production d'une préparation tolérante d'immunoglobuline G intraveineuse

(43) Veröffentlichungstag der Anmeldung: 25.09.1991
(73) Patentinhaber: BIOTEST PHARMA GMBH, D-63303 Dreieich (DE)
(72) Erfinder: Kothe, Norbert, Dr. Dipl.-Chem., D-6242 Kronberg 1 (DE); Rudnick, Dieter, Dr. Dipl.-Chem., D-6072 Dreieich (DE); Piechaczek, Detlef, Dr. Dipl.-Chem., D-6115 Münster (DE); Klein, Herwald, Dr., D-6108 Weiterstadt (DE); Rohm, Detlef, Dr. Dipl.-Biol., D-64367 Mühltal (DE); Kloft, Michael, Dr. Dipl.-Chem., D-6100 Darmstadt (DE)
(74) Vertreter: Strych, Werner Maximilian Josef, Dr.

(56) Entgegenhaltungen:
- EP-A- 0 013 901
- EP-A- 0 234 405
- EP-A- 0 268 973
- EP-A- 0 338 229
- EP-A- 0 346 217
- EP-A- 0 352 500
- EP-A2- 0 268 973
- DE-A- 3 310 150
- US-A- 4 164 495
- JOURNAL OF IMMUNOLOGICAL METHODS vol. 65, 1983, HOLLAND, Seiten 269 - 271; Russo,C. et al: "Purification of IgG monoclonal antibody by caprylic acidprecipitation"
- CHEMICAL ABSTRACTS, vol. 103, no. 13, 30 September 1985, Columbus, Ohio, USA; Goheen,S.C. et al: "Purification of human serum gamma globulins by hydrophobicinteraction high-performance liquid chromatography" Seite 458; linke Spalte; ref. no. 103013
- ARCHIVES OF BIOCHEMISTRY AND BIOPHYSICS, vol. 89, 1960, Seiten 218 - 220; Chanutin A. et al: "The precipitation of plasma proteins by short-chain fattyacids"
- PREPARATIVE BIOCHEMISTRY, vol. 14, no. 1, 1984, Seiten 1 - 17; Habeeb, A. et al: "Preparation of human immunoglobulin by caprylic acid precipitation"

## Beschreibung

Die Erfindung betrifft das in den Patentansprüchen beschriebene Verfahren zur Herstellung eines intravenös verträglichen Immunglobulin-G-Präparates.

Aus menschlichem Plasma gewonnene Immunglobulin-Präparate werden seit vielen Jahren für die Therapie angeborener und erworbener Immunmangel-Krankheiten eingesetzt. Ursprünglich wurde das Immunglobulin-G durch fraktionierte Fällung mit Ethanol aus Humanplasma gewonnen. Die auf diese Art hergestellten Präparate waren jedoch nur intramuskulär anwendbar, da bei intravenöser Anwendung schwerste Nebenwirkungen auftraten.

Bei der intramuskulären Applikation ist die Dosierung limitiert. Um ausreichende Plasmaspiegel an Immunglobulinen, wie sie für eine effektive Therapie notwendig sind, zu erreichen, war es deshalb wünschenswert, intravenös verträgliche Immunglobulin-G-Präparate herzustellen.

Bisher sind hierfür verschiedene Methoden beschrieben. So wurden beispielsweise die IgG-Moleküle mit Enzymen gespalten oder mit chemischen Reagenzien modifiziert. Andere Verfahren beschreiben den Einsatz von Fällungs-, Adsorptions- und Chromatographie-Schritten, um intravenös verträgliche Präparate herzustellen.

Eine Übersicht über die derzeit angewandten Methoden ist in Krankenhauspharmazie, 6. Jahrgang, Nr. 5, 1985, S. 226-231 (1) und Der Bayerische Internist 9, 1 1989, S. 36-44 (2) gegeben.

Alle dort beschriebenen Präparate sind zwar intravenös applizierbar, weisen jedoch je nach Art der Herstellung verschiedene Nachteile auf. So haben die enzymatisch gespaltenen Immunglobuline eine stark verkürzte intravasale Halbwertszeit und können die zur effektiven Infektabwehr notwendige Komplementaktivierung nicht auslösen. Auch die chemische modifizierten Präparate können nicht alle biologischen Funktionen erfüllen. Auch hier wird eine verkürzte intravasale Halbwertszeit sowie eine gestörte Komplementaktivierung beschrieben.

Um die obengenannten Nachteile auszuräumen, wurden daher verbesserte Methoden zur Herstellung eines iv-verträglichen, nicht modifizierten IgG-Präparates entwickelt.

So offenbart beispielsweise die DE-A 30 39 855 eine Filtration an einer Polysulfonmembran, um insbesondere polymere Substanzen (Aggregate) zu entfernen.

In der DE-A 36 41 115 wird vorgeschlagen, eine Reinigung mittels Polyethylenglykolen zur Entfernung von Aggregaten vorzunehmen.

Mit beiden Verfahren gelingt es jedoch nicht, proteolytische Aktivitäten bzw. vasoaktive Substanzen zu entfernen. Von diesen Substanzen, insbesondere dem Prekallikrein-Aktivator (PKA), Prekallikrein, Kininogen und Kallikrein, die in Immunglobulin-G-Präparaten vorhanden sein können, ist jedoch bekannt, daß sie als Ursache für schwerwiegende Nebenwirkungen, insbesondere als Auslöser für hypotensive Reaktionen, fungieren können (vgl. M.F. Makula et al., Develop. biol. Standard, Vol. 67, 257-265, 1987, W.K. Bleeker et al., Vox. Sang. 52, 281-2190, 1987).

Versuche, proteolytische Enzyme durch Adsorption mittels Bentonit, Kieselgel, Bariumsulfat, Aktivkohle sowie Affinitätsaustauschern zu entfernen, führten jedoch nicht zu einer spezifischen Abtrennung aller störenden Proteasen, da je nach Herstellungsmethode von Immunglobulin-G-Präparaten darin mindestens noch unterschiedliche Mengen an Prekallikrein, Kininogen und Kallikrein unter Anwendung immunologischer und enzymatischer Meßmethoden festgestellt wurden, selbst wenn keine signifikante Menge an Prekallikrein-Aktivator (PKA) nachgewiesen wurde.

Die Verabreichung solcher Präparate, enthaltend die genannten Substanzen, führten, obwohl diese Präparate in anderen Anwendungen sehr gut i.v. verträglich sind, insbesondere bei immunsupprimierten Patienten zu schweren Nebenwirkungen. So kann - auch bei Nichtnachweisbarkeit des PKA - bei Anwesenheit von Prekallikrein das Kallikrein freigesetzt werden, das aus hochmolekularem Kininogen das vasoaktive Bradykinin freisetzt. Dies bewirkt in Abhängigkeit der Konzentration einen mehr oder weniger ausgeprägten Blutdruckabfall. Vor allem bei Patienten, deren Inhibitorpotential gestört ist, wie bei einer Immunsuppression, kommt dies zum Tragen.

Für ein allgemein anwendbares intravenös verträgliches Immunglublin-G-Präparat besteht daher die Forderung, daß es frei ist
a) von Aggregaten, so daß nur eine niedrige unspezifische Aktivierung des Komplementsystems vorliegt,
b) von proteolytischen Enzymen
c) von vasoaktiven Substanzen des Gerinnungs- und Kininsystems, wie Prekallikrein-Aktivator (PKA), Prekallikrein, Kininogen, Kallikrein, Faktor XI und Faktor XII und daß es
d) biologisch intakt ist, d.h. keine Strukturveränderungen des Fc-Teils des Immunglobulin-G während des

Herstellungsprozesses erfolgen und keine chemische Modifizierung angewendet wird.

Der vorliegenden Erfindung liegt daher die Aufgabe zugrunde, ein bei allen Patienten-Kollektiven einsetzbares, intravenös verträgliches Immunglobulin-G-Präparat, das frei von Aggregaten, vasoaktiven Substanzen und proteolytischer Aktivität ist, aus einem Immunglobulin-haltigen Ausgangsmaterial, wie einer von Gerinnungsfaktoren befreiter Immunglobulin-G-haltigen Serumfraktion (Cohn-Paste II oder II/III), bereitzustellen.

Diese Aufgabe wird erfindungsgemäß dadurch gelöst, daß das Immunglobulin-G-haltige Ausgangsmaterial ausgewählt aus einer durch Cohn-Fraktionierung gewonnene Gammaglobulin-Fraktion (Cohn-Paste II oder II/III) oder einer aus Serum chromatographisch vorgereinigte Roh-Immunglobulin-G-haltige Lösung einer Behandlung mit 0,4-1,5 Vol.-% Oktansäure und einer Chromatographie unterzogen wird.einer Behandlung mit 0,4-1,5 Vol.-% Oktansäure und einer Chromatographie unterzogen wird.

Die Anwendung von Oktansäure ist in der Literatur beschrieben (Biochemistry and Biophysics 134, 279-284, 1969). Dabei wird Oktansäure in Konzentrationen von etwa 6,8 Vol.-% zur Fällung von Proteinen aus Plasma oder Serum eingesetzt, während IgG im Überstand bleibt.

Überraschenderweise wurde nun gefunden, daß Oktansäure in Konzentrationen von 0,4 bis 1,5 Vol.-% und bevorzugt von 0,8 bis 1,0 Vol.-% spezifisch proteolytische Enzyme sowie Verbindungen, die vasoaktive Reaktionen auslösen, wie z.B. Prekallikrein, Kallikrein und Kininogen, aus vorgereinigten IgG-Lösungen entfernen kann, ohne daß eine nennenswerte Proteinpräzipitation erfolgt.

Aus einer solchermaßen gereinigten Immunglobulin-G-haltigen Lösung kann anschließend die Oktansäure durch Zentrifugation, vorzugsweise jedoch durch Filtration unter Zusatz von Calciumionen entfernt werden.

Die Wirksamkeit der Oktansäurebehandlung hinsichtlich der Entfernung von proteolytischen Enzymen sowie vasoaktiven Substanzen aus Immunglobulin-G-haltigen Lösungen¹⁾ ist in den nachfolgenden Tabellen 1 und 2 aufgezeigt.

**Tabelle 1**

| Oktansäurekonzentration (V/V %) | Proteolytische Aktivität (U/l) |
|---|---|
| 0 | 850 |
| 0,4 | 23 |
| 0,6 | 1 |
| 0,8 | 0,0 |
| 1,0 | 0,0 |
| ¹⁾ Ausgangsmaterial: lmmunglobulin-G-Röhfraktion aus Cohn-Prozeß (wie in Beispiel 1) | |

**Tabelle 2**

| Oktansäurekonzentration (V/V %) | Prekallikrein* | Kallikrein* |
|---|---|---|
| 0 | + + | + + |
| 0,8 | - | - |
| 1,0 | - | - |
| 1,5 | - | - |

| | | |
|---|---|---|
| * Bestimmung durch Immundiffusion nach Ouchterlony 1) Ausgangsmaterial: Immunglobulin-G-Rohfraktion aus chromatographischen Prozessen wie in Beispiel 5 | | |

Wie obige Ergebnisse zeigen, werden erfindungsgemäß vasoaktive Substanzen und proteolytische Enzyme aus den Immunglobulin-G-haltigen Lösungen entfernt.

In einem nachfolgenden Schritt kann die Immunglobulin-G-haltige Lösung durch Chromatographie von aggregierten IgG-Molekülen befreit werden.

Hierfür eignen sich Chromatographie-Phasen auf Kieselgel oder Polymerbasis, wobei sowohl Kationenals auch Anionenaustauscher verwendet werden können.

Bevorzugt werden solche Chromatographie-Phasen verwendet, die für HPLC (Hochleistungsflüssigkeitschromatographie) geeignet sind, da mit diesem Verfahren große Mengen Immunglobulin-G in kurzer Zeit wirtschaftlich hergestellt werden können.

Bei Verwendung von Kationenaustauschern, wie z.B. CM-Accell^{(R)}, SP-Spherodex^{(R)}, SP-Trisacryl-LS^{(R)} oder Fraktogel-TSK-SP 650^{(R)}, wird die Salzkonzentration des Eluenten auf 50-200 mMol Natriumchlorid und der pH-Wert auf 4,0-6,0 eingestellt. Unter diesen Bedingungen werden die Aggregate an den Träger gebunden, die gereinigte IgG-Fraktion passiert ungebunden die Matrix.

Nach der gleichen Methode gelingt die Entfernung von Aggregaten aus Immunglobulin-G-Lösungen auch mit Anionenaustauschern, wie z.B. QMA-Accell^{(R)} und DEAE-Spherosil^{(R)},wenn man eine lonenkonzentration von 0-50 mMol Natriumchlorid und einen pH-Wert von 6,0 bis 8,0 einstellt.

Darüberhinaus zeigte sich, daß mittels der genannten Chromatographie-Phasen, insbesondere bei Verwendung von Kationenaustauschern oder Hydrophoben-Phasen auch vasoaktive Substanzen entfernt werden können. Bevorzugt werden hierfür Chromatographie-Phasen auf Kieselgelbasis wie CM-Accell^{(R)}, SP-Spherodex-M^{(R)} und Polypropyl A^{(R)} oder auf der Basis synthetischer Polymere hergestellte Phasen wie SP-Trisacryl-LS^{(R)} und CM-Trisacryl-LS^{(R)} verwendet.

Im Falle eines Kationenaustauschers wird die lonenstärke und der pH-Wert des Eluenten so gewählt, daß die gereinigte Immunglobulin-G-Fraktion die Säule ungebunden passiert und die Verunreinigungen an der Chromatographie-Phase gebunden bleiben.

Im Falle von CM-Accell^{(R)} verwendet man beispielsweise Puffer mit einer Salzkonzentration von 50 - 200 mMol Natriumchlorid bei einem pH-Wert von 4,0 bis 6,0.

Soll die Reinigung über hydrophobe Interaktions-Chromatographie an Polypropyl A^{(R)} durchgeführt werden, so wird die IgG-haltige Lösung mit einem hochmolaren Ammoniumsulfat-Puffer an die Phase gebunden, die Verunreinigung durch Nachwaschen entfernt und anschließend die Immunglobulin-G-Fraktion mit einem linearen abfallenden lonenstärke-Gradienten eluiert.

Die Wirksamkeit der chromatographischen Entfernung vasoaktiver Substanzen aus Immunglobulin-G-haltigen Lösungen ist in nachfolgender Tabelle 3 aufgezeigt.

**Tabelle 3**

| IgG-Fraktion | Prekallikrein* | Kallikrein* |
|---|---|---|
| unbehandelt** | ++ | ++ |
| nach SP-Chromatographie | - | - |
| nach CM-Chromatographie | - | - |
| nach Chromatographie an | | |
| Polypropyl A^{(R)} | - | - |

| | | |
|---|---|---|
| * Bestimmung durch Immundiffusion nach Ouchterlony | | |
| ** Ausgangsmaterial: Immunglobulin-G-Rohfraktion aus chromatographischen Prozessen wie in Beispiel 5 | | |

Somit können hochreine iv-verträgliche Immunglobulin-G-Präparate durch die erfindungsgemäße Behandlung mit Oktansäure und/oder Chromatographie hergestellt werden. Dabei kann als Ausgangsmaterial eine durch übliche Cohn-Fraktionierung gewonnene gamma-Globulin-Fraktion (Cohn-Paste II oder Paste II/III oder entsprechende Lösungen), wie sie bei der großtechnischen Herstellung von Humanalbumin in großen Mengen anfällt, eingesetzt werden. Ferner ist das erfindungsgemäße Verfahren auch auf chromatographisch isolierte IgG-Fraktionen anwendbar. Diese Ausgangsmaterialien können sowohl von normalen Spenderpools als auch bevorzugt von ausgewählten Spendern mit hohen Antikörpertitern gegen virale, bakterielle oder zelluläre Antigene gewonnen werden.

Vorzugsweise werden als Ausgangsmaterial des erfindungsgemäßen Verfahrens Immunglobulin-G-haltige Rohfraktionen aus chromatographischen oder Cohn-Prozessen, die einem Sterilisationsverfahren unterzogen wurden, eingesetzt. Chromatographie-Fraktionen enthalten üblicherweise etwa 0,5 bis 1,5% Aggregate, die bevorzugt an Kationenaustauschern wie z.B. CM-Accell^{(R)} vollständig abgetrennt werden können. Geht man von einer Cohn-Paste II oder II/III aus, welche bis zu 5% Aggregate enthalten kann, gelingt die vollständige Entfernung derselben erfindungsgemäß mit Hilfe von CM-Accell^{(R)} oder QMA-Accell^{(R)}. Die Entfernung vasoaktiver Substanzen kann wie oben beschrieben gleichfalls chromatographisch erfolgen.

Sofern proteolytische Enzyme vorhanden sind, werden diese zuvor mittels Oktansäure-entfernt, was jedoch im Falle von Immunglobulin-G-haltigen Lösungen, die sich durch eine hohe Reinheit auszeichnen, entfallen kann.

Da die im erfindungsgemäßen Verfahren eingesetzten Plasma- oder Serum-Fraktionen potentiell mit humanpathogenen Viren, wie Hepatitis A, B, Non A, Non B und HIV, kontaminiert sein können, wird es bevorzugt, die Immunglobulin-G-haltige Lösung einer Sterilisation zu unterwerfen. Hierzu können Behandlungen, vorzugsweise vor dem Chromatographie-Schritt, mit β-Propiolacton, TNBP/Tween, TNBP/Nacholat, TNBP, gegebenenfalls in Kombination mit UV-Bestrahlung angewendet werden. Besonders bevorzugt ist dabei die Sterilisation mittels 0,03-0,1 Vol.-% β-Propiolacton in Kombination mit einer UV-Bestrahlung.

Die auf diese Weise erfindungsgemäß hergestellten Produkte können flüssig oder lyophilisiert gelagert werden.

Der Vorteil des erfindungsgemäßen Verfahrens liegt darin, daß auf schnelle und einfache Weise verschiedenartigste Immunglobulin-G-haltige Ausgangsmaterialien so gereinigt werden können, daß die erhaltenen reinen Produkte nebenwirkungsfrei bei allen Patienten-Kollektiven und insbesondere bei immunsupprimierten Patienten intravenös eingesetzt werden können.

Im folgenden werden die erfindungsgemäß hergestellten Präparate im Vergleich zu Produkten gemäß Stand der Technik in Tierexperimenten auf ihre Verträglichkeit hin untersucht.

Dabei wurde zum einen ein Rattenmodell (vgl. Bleeker et al., Vox Sang. 52, 281-290 (1987)) verwendet, worin die Beeinflussung des Blutdrucks und der Herzfrequenz durch vasoaktive Substanzen, wie sie in herkömmlichen Produkten vorhanden sein können, untersucht wurde.

Die Ergebnisse sind in der nachfolgenden Tabelle 4 zusammengefaßt.

**Tabelle 4**

| Präparat | %Abweichung Blut-druck | Herz- frequenz | Vasoaktive Substanzen | | |
|---|---|---|---|---|---|
| | | | Prekallikrein¹⁾ | Kallikrein¹⁾ | Kininogen¹⁾ |
| 1²⁾(Vgl.) | -30 | +6 | + | + | + |
| 2²⁾ (Vgl.) | -42 | -8 | ++ | + | + |
| 3²⁾ (Vgl.) | -32 | +2 | ++ | + | + |
| 4*(Erf.) | -2 | -4 | - | - | - |
| 5* (Erf.) | -3 | +3 | - | - | - |
| 6** (Erf.) | -4 | +1 | - | - | - |
| 7** (Erf.) | 0 | +1 | - | - | - |
| SE aus 7*** | -13 | +10 | ++ | + | + |

| | | | | | |
|---|---|---|---|---|---|
| 1) durch Immundiffusion nach Ouchterlony bestimmt. | | | | | |
| 2) Immunglobulin-G-Rohfraktion aus chromatographischen Prozessen wie in Beispiel 5 | | | | | |
| * hergestellt gemäß Beispiel 8 | | | | | |
| ** hergestellt gemäß Beispiel 9 | | | | | |
| *** Säuleneluat des Präparates 7 als Positiv-Kontrolle (enthaltend vasoaktive Substanzen) | | | | | |

Bis zu einem Blutdruckabfall von -10% gilt die Lösung als verträglich.

Wie die Ergebnisse dieses Versuchs zeigen, bewirken erfindungsgemäß über einen lonenaustauscher gereinigte Präparate so gut wie keine Schwankungen bezüglich des Blutdrucks und der Herzfrequenz, was ihre höhere Verträglichkeit im Vergleich zu herkömmlichen Präparaten dokumentiert.

In einem weiteren Experiment wurde im Hundemodell nach M.F.Makula et al., Develop. biol. Standard Vol. 67, 257-265, 1987, die Verträglichkeit erfindungsgemäß hergestellter Immunglobulin-G-Lösungen im Vergleich zu herkömmlich bereiteten Präparaten untersucht.
Nach diesem Modell wird vorwiegend die Beeinflussung durch polymere Stoffe (Aggregate) durch Messung der Veränderung im Herzzeitvolumen bestimmt.

Die Ergebnisse sind in nachfolgender Tabelle 5 zusammengefaßt.

**Tabelle 5**

| Präparat | %-Abweiweichung Herzzeit-volumen | Aggregate % HPSEC | Vasoaktive Substanzen¹⁾ | | |
|---|---|---|---|---|---|
| | | | Prekallikrein | Kallikrein | Kininogen |
| 8²⁾ (Vgl.) | -50 | 1,1 | + | + | + |
| 9³⁾ (Vgl.) | -49 | 1,91 | ++ | + | + |
| 10* (Erf.) | 0 | 0 | - | - | - |
| 11**(Erf.) | -13 | 0 | - | - | - |
| 12***(Erf.) | 0 | 0 | - | - | - |

| | | | | | |
|---|---|---|---|---|---|
| 1) durch Immundiffusion nach Ouchterlony bestimmt | | | | | |
| 2) Immunglobulin-G-Rohfraktion aus chromatographischen Prozessen wie in Beispiel 5 | | | | | |
| 3) Immnglobulin-G-Rohfraktion aus Cohn-Prozess wie in Beispiel 1 | | | | | |
| * hergestellt gemäß Beispiel 1 vorliegender Erfindung | | | | | |
| ** hergestellt gemäß Beispiel 8 vorliegender Erfindung | | | | | |
| *** hergestellt gemäß Beispiel 5 vorliegender Erfindung | | | | | |

Bis zu einer Abweichung im Herzzeitvolumen von -15% gilt die Lösung als verträglich.

Wie auch dieser Versuch zeigt, sind die erfindungsgemäß hergestellten Präparate von einer höheren Reinheit und damit einer Nebenwirkungsfreiheit, wie sie die bisher bekannten Produkte nicht aufweisen. Durch die vollständige Entfernung von Aggregaten, proteolytischer Substanzen sowie vasoaktiver Verbindungen können Immunglobulin-G-Präparate hergestellt werden, die auch insbesondere bei immunsupprimierten Patienten angewendet werden können.

Die Erfindung wird durch nachstehende Beispiele erläutert.

### Beispiel 1

5 kg Cohn-Paste II werden in 45 I eines 0,1 M NaCl, 0,075 M Natriumacetat, pH 5,5 Puffers gelöst. Man versetzt mit 10 ml Oktansäure pro I Lösung und rührt 1 Stunde bei Raumtemperatur. Danach wird die Lösung auf 0°C abgekühlt, mit 12,3 g Calciumacetat pro I Lösung versetzt, 3 Stunden gerührt und anschließend filtriert. Das klare Filtrat wird gegen das 10-fache Volumen eines 0,022 molaren Tris-Puffers, pH 7,0, diafiltriert und anschließend auf eine Proteinkonzentration von 4% Protein eingestellt. Die 4%ige Proteinlösung wird bei pH 7,2 mit 0,05% β-Propiolacton versetzt und bei konstantem pH-Wert 12 Stunden gerührt. Danach verdünnt man mit 0,022 M Tris-Puffer auf einen Proteingehalt von 1% und bestrahlt diese Lösung in einer UV-Durchflußapparatur mit einer Durchflußgeschwindigkeit von 20 I pro Stunde.

Nach Zugabe von 2 g Kochsalz pro Liter Lösung wird sterilfiltriert. Die chromatographische Reinigung erfolgt mit einer präparativen HPLC-Anlage, die mit einer 5 I HPLC-Säule, gefüllt mit QMA-Accell, bestückt ist.

Nach Äquilibrierung der Säule mit einem 0,022 M Tris/0,035 M NaCl, pH 7,0, Puffer wird die sterilisierte Immunglobulin-Lösung in Portionen von 15 I chromatographiert, wobei die Verunreinigungen und Aggregate an die Chromatographie-Phase gebunden werden. Die hochreine IgG-Fraktion passiert ungebunden die Säule. Zur Aufreinigung der Gesamtmenge sind 8 Zyklen notwendig. Ein Zyklus inklusive Proteinauftrag, Elution und Regeneration erfordert 2 Stunden.

Die gereinigte IgG-Fraktion wird mit Hilfe einer Ultrafiltrationsanlage (cut off 10.000 D) auf 50 g/l ankonzentriert und gegen das 10-fache Volumen einer 0,45%igen Kochsalzlösung diafiltriert. Man fügt 0,15 m/l Glycin zu und führt eine Sterilfiltration durch.

Die so gewonnene Immunglobulin-G-Lösung hat folgende Eigenschaften:

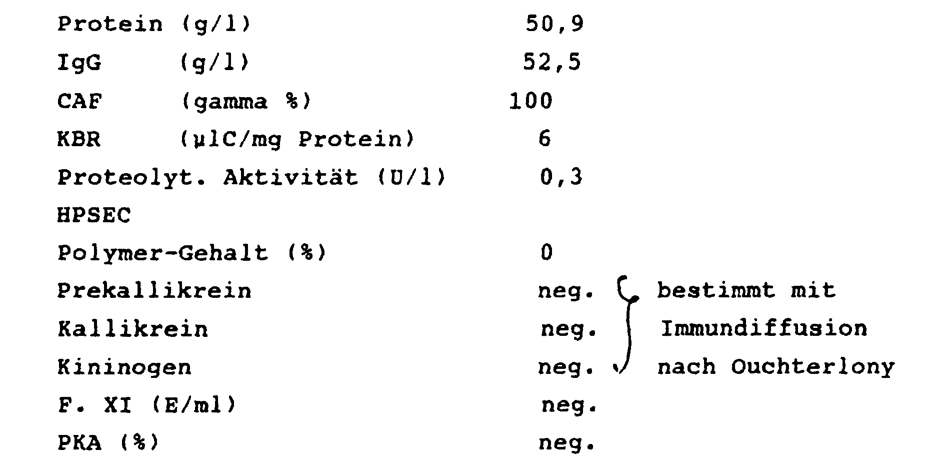

### Beispiel 2

Man verfährt wie in Beispiel 1.

Der Chromatographie-Schritt wird unter Normaldruck an einer 5 I Säule, gefüllt mit QMA-Accell, durchgeführt. In einem Zyklus können 15 I der sterilisierten Immunglobulin-G-Lösung aufgereinigt werden. Die Zyklusdauer beträgt hier 4 1/2 Stunden.

Die weiteren Schritte werden wie in Beispiel 1 beschrieben durchgeführt.

Die Immunglobulin-G-Lösung hat folgende Eigenschaften:

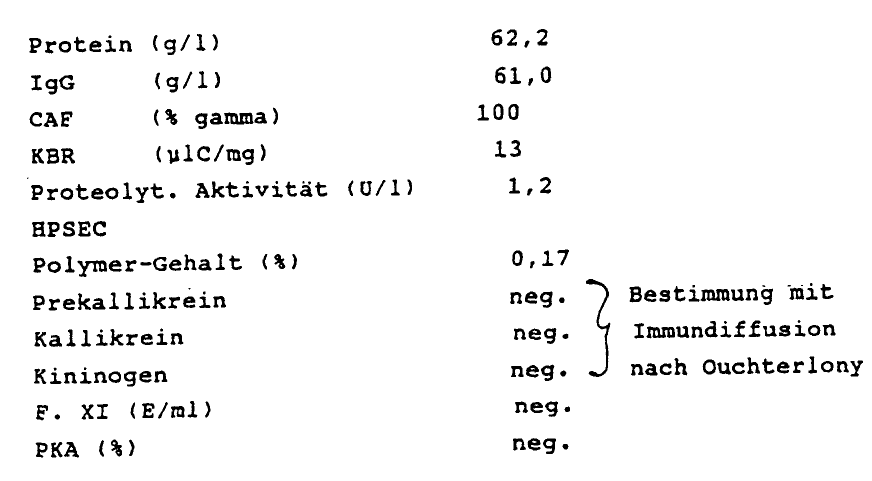

### Beispiel 3

Man verfährt wie in Beispiel 2.

Anstelle der 5 I-Säule mit QMA-Accell verwendet man DEAE-Spherosil-LS als Chromatographie-Phase. Die damit erhaltene Immunglobulin-G-Lösung hat folgende Eigenschaften.

| | |
|---|---|
| Protein (g/l) | 48,0 |
| IgG (g/l) | 47,4 |
| CAF (% gamma) | 100 |
| KBR (µlC/mg) | 16 |

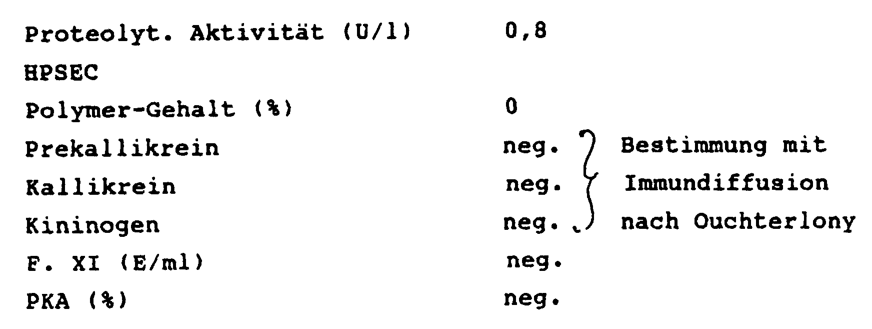

### Beispiel 4

Man verfährt wie in Beispiel 1.

Das klare Filtrat nach der Oktansäurebehandlung wird gegen das 10-fache Volumen mit einem 0,025 M Natriumacetat-Puffer, pH 7,0, diafiltriert und auf 4 % Protein eingestellt.

Danach führt man die β-Propiolacton-Behandlung mit UV-Bestrahlung analog Beispiel 1 durch. Die sterilisierte Lösung wird mit 2 g Kochsalz pro Liter Lösung versetzt, der pH-Wert auf pH 5,0 eingestellt und sterilfiltriert.

Als Chromatographie-Phase findet CM-Accell Verwendung. An die mit 0,025 M Natriumacetat, 0,035 M NaCl, pH 5,0, äquilibrierte Säule bindet das IgG und wird anschließend mit 0,025 M Natriumacetat, 0,200 M NaCI, pH 5,0, eluiert.

Die weitere Aufarbeitung erfolgt wie in Beispiel 1 beschrieben.

Die Immunglobulin-G-Lösung hat folgende Eigenschaften:

| | |
|---|---|
| Protein (g/l) | 50,0 |
| IgG (g/l) | 51,03 |
| CAF (% gamma) | 100 |
| KBR (µlC/mg Protein) | 11 |

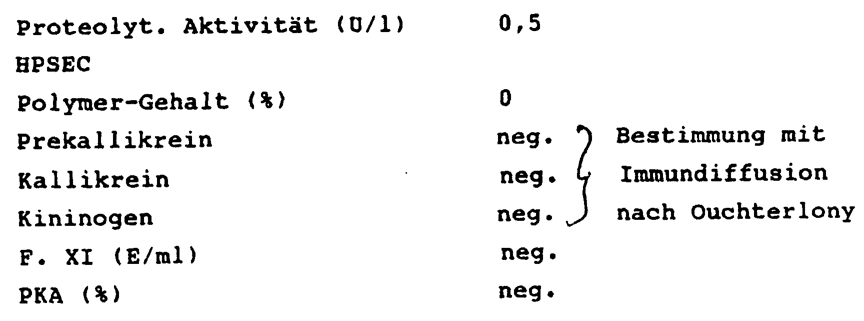

### Beispiel 5

Fresh frozen Humanplasma wird bei +4°C aufgetaut und das Kryopräzipitat durch Zentrifugation abgetrennt. Die Entfernung der Gerinnungsfaktoren des PPSB-Komplexes erfolgt mittels Batch-Adaption an DEAE-Sephadex A 50. Nach Abtrennung des Ionenaustauschers wird der Überstand mit 9 Vol.-% Alkohol bei pH 5,3 versetzt. Der Niederschlag wird durch Zentrifugation entfernt. Das so erhaltene, von Gerinnungsfaktoren befreite Plasma wird mit Hilfe einer Gelausschlußchromatographie (Sephadex G-25) auf ein lonenmilieu von 0,022 M Tris/HCl, pH 7,5, eingestellt.

An einer Säule, gefüllt mit DEAE-Trisacryl-LS und äquilibriert mit dem gleichen Puffer, wird das IgG von den übrigen Plasmaproteinen abgetrennt. Man erhält das Roh-IgG in der Durchlauffraktion, die durch Ultrafiltration auf 40 g/l Protein ankonzentriert wird.

Man fügt pro Liter Lösung 5,8 g NaCI, 10,2 g Natriumacetat zu, stellt den pH-Wert auf pH 5,5 ein und versetzt mit 10 ml Oktansäure. Nach einer Reaktionszeit von einer Stunde bei Raumtemperatur und Zugabe von 12,3 g Calciumacetat pro Liter Lösung wird 3 Stunden bei 0°C gerührt.

Die Reaktionsmischung wird filtriert, gegen das 10-fache Volumen mit 0,025 M Na-Acetat/0,140 M NaCI Puffer, pH 7,0, diafiltriert und auf 4 % Protein eingestellt.

Die Sterilisation mit β-Propiolacton und UV-Bestrahlung wird analog Beispiel 1 durchgeführt.

Der pH-Wert der sterilisierten IgG-Lösung wird auf pH 5,0 eingestellt.

Zur Chromatographie setzt man eine Säule ein, die mit CM-Accell gefüllt und mit 0,025 M Na-Acetat, 0,140 M NaCI, pH 5,0, äquilibriert ist. Pro Liter Chromatographie-Phase werden 9 I der sterilisierten IgG-Lösung aufgegeben. Unter diesen Bedingungen binden die Verunreinigungen und Aggregate an die Phase, die hochreine IgG-Fraktion passiert die Säule.

Die weitere Aufarbeitug erfolgt wie in Beispiel 1 beschrieben.

Die Immunglobulin-Lösung hat folgende Eigenschaften:

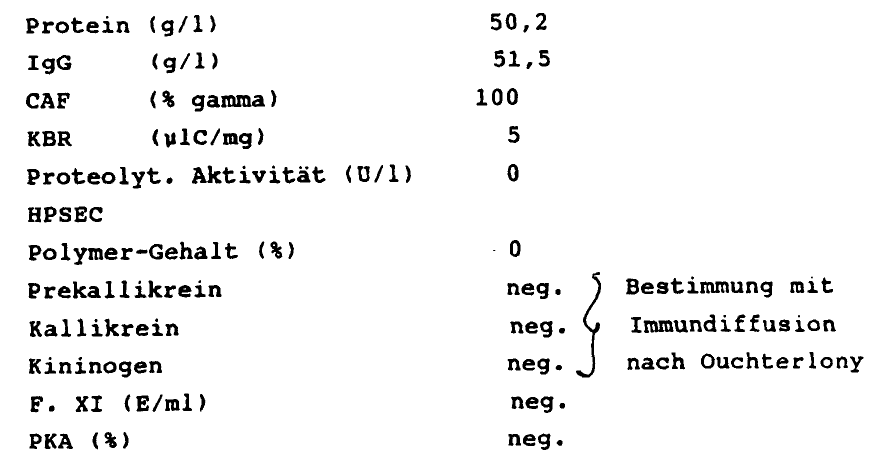

### Beispiel 6

Man verfährt wie in Beispiel 5.

Die chromatographisch isolierte, Oktansäure-behandelte und sterilisierte IgG-Lösung wird auf pH 5,0 eingestellt und sterilfiltriert.

Die anschließende Chromatographie erfolgt an einer Säule, die mit SP-Trisacryl-LS gefüllt und mit 0,025 M Na-Acetat, 0,150 M NaCl, pH 5,0, äquilibriert ist. Pro Liter Chromatographie-Phase werden 3 I der sterilisierten IgG-Lösung aufgegeben.

Man verfährt weiter wie in Beispiel 5 beschrieben.

Die Immunglobulin-Lösung hat folgende Eigenschaften:

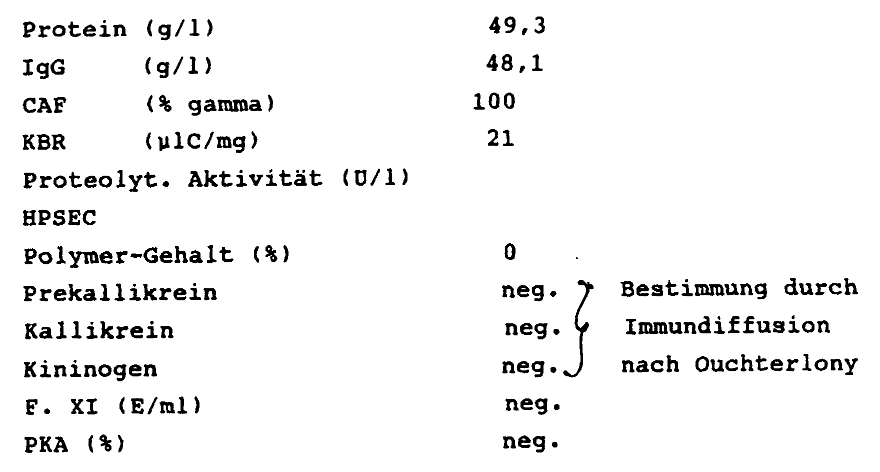

### Beispiel 7

Man verfährt wie in Beispiel 5.

Die chromatographisch isolierte und Oktansäure-behandelte IgG-Lösung wird gegen das 10-fache Volumen mit 0,1 M Phosphat-Puffer, pH 7,0, diafiltriert und auf 4 % Protein eingestellt.

Die Sterilisation mit β-Propiolacton und UV-Bestrahlung wird analog Beispiel 1 durchgeführt.

Die sterilisierte IgG-Lösung mit einem Proteingehalt von 1% versetzt man mit festem Ammonsulfat auf eine Endkonzentration von 1 Mol Ammonsulfat pro Liter und führt eine Sterilfiltration durch.

Eine HPLC-Säule, gefüllt mit Polypropyl-A, wird mit 1 M Ammonsulfat, 0,1 M Phosphat, pH 7,0, Puffer äquilibriert. Zwei Liter der eingestellten IgG-Lösungen werden pro Liter Chromatographie-Phase aufgegeben. Unter diesen Bedingungen bindet das IgG an die Phase, die Verunreinigungen werden mit dem Äquilibrierpuffer ausgewaschen. Die IgG-Fraktion eluiert man mit 0,1 M Phosphat, pH 7,0.

Die Weiterverarbeitung erfolgt gemäß Beispiel 1.

Die Immunglobulinlösung hat folgende Eigenschaften:

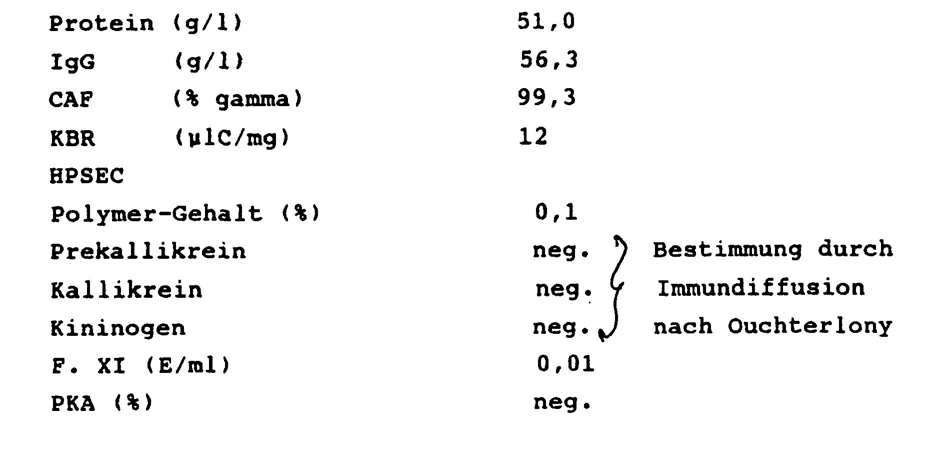

### Beispiel 8

Man verfährt wie in Beispiel 5.

Die auf 40 g/l ankonzentrierte IgG-Fraktion wird mit 8,2 g NaCI und 2,05 g Na-Acetat pro Liter Lösung versetzt und analog Beispiel 1 einer Sterilisation mit β-Propiolacton und UV-Bestrahlung unterzogen.

Der pH-Wert der sterilisierten IgG-Lösung wird auf pH 5,0 eingestellt.

Diese Lösung wird an einer Säule, die mit CM-Accell gefüllt und mit 0,025 M Na-Acetat, 0,140 M NaCI, pH 5,0 äquilibriert ist, chromatographiert.

Die hochreine IgG-Fraktion passiert die Säule, die Verunreinigungen und Aggregate werden an die Phase gebunden.

Die weitere Aufarbeitung erfolgt wie in Beispiel 1 beschrieben.

Die Immunglobulin-Lösung hat folgende Eigenschaften:

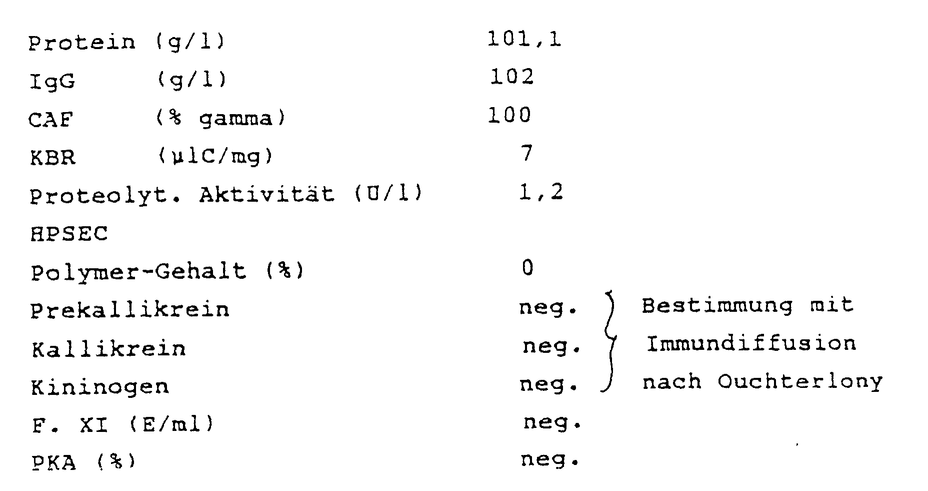

### Beispiel 9

Man verfährt wie in Beispiel 5.

Die chromatographisch isolierte und sterilisierte IgG-Lösung wird pro Liter Lösung mit 9 g NaCl und 2,05 g Na-Acetat versetzt, der pH-Wert wird auf pH 5,0 eingestellt.

Die Chromatographie erfolgt an einer Säule, die mit SP-Trisacryl-LS gefüllt und mit 0,025 M Na-Acetat, 0,150 M NaCl, pH 5,0 äquilibriert ist.

Man verfährt weiter wie in Beispiel 5 beschrieben.

Die Immunglobulin-haltige Lösung hat folgende Eigenschaften:

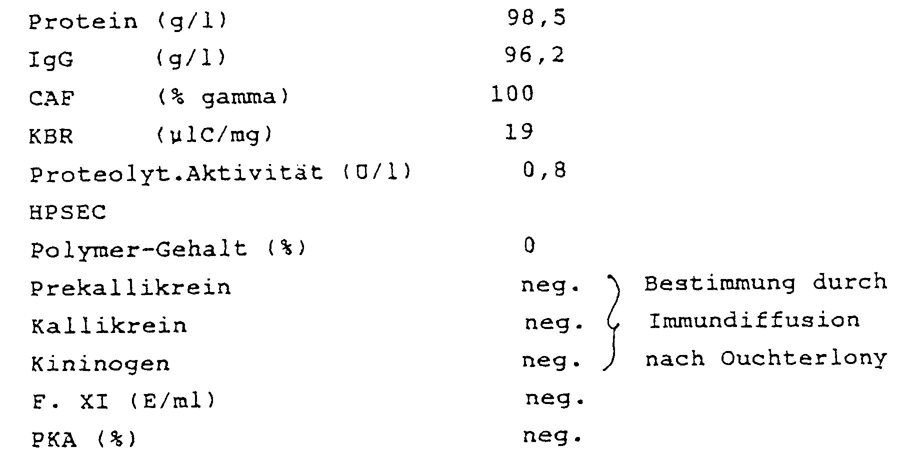

## Patentansprüche

1. Verfahren zur Herstellung einer intravenös verträglichen polyklonalen Immunglobulin-G-Lösung aus einem von Gerinnungsfaktoren befreiten Immunglobulin-G-haltigen Ausgangsmaterial, ausgewählt aus einer durch Cohn-Fraktionierung gewonnene Gammaglobulin-Fraktion (Cohn-Paste II oder II/III) oder einer aus Serum chromatographisch vorgereinigte Roh-Immonglobulin-G-haltige Lösung ,
**dadurch gekennzeichnet, daß** man
a) das in üblicher Weise aufgearbeitete Ausgangsmaterial mit 0,4 bis 1,5 Vol.-% Oktansäure behandelt, die Lösung anschließend reinigt und
b) die so erhaltene Lösung an Anionen- oder Kationenaustauschern oder über eine hydrophobe Interaktions-Chromatographie chromatographiert.

2. Verfahren gemäß Anspruch 1, **dadurch gekennzeichnet, daß** die Immunglobulin-G-haltige Lösung in Schritt a) mit 0,8 bis 1 Vol.-% Oktansäure behandelt wird.

3. Verfahren nach einem der Ansprüche 1 oder 2, **dadurch gekennzeichnet, daß** die Chromatographie an für Hochleistungsflüssigkeitschromatographie (HPLC) geeigneten Phasen erfolgt.

4. Verfahren nach einem der Ansprüche 1 bis 3, **dadurch gekennzeichnet, daß** als Anionenaustauscher QMA-Accell^{(R)} oder DEAE-Spherosil^{(R)} verwendet werden.

5. Verfahren nach einem der Ansprüche 1 bis 3, **dadurch gekennzeichnet, daß** als Kationenaustauscher CM-Accell^{(R)}, Sp-Spherodex^{(R)}, SP-Trisacryl-LS^{(R)} oder Fraktogel TSK SP 650^{(R)} verwendet werden.

6. Verfahren nach einem der Ansprüche 1 bis 3, **dadurch gekennzeichnet, daß** die Chromatographie an Polypropyl A^{(R)} als hydrophobe Phase erfolgt.

7. Verfahren nach einem der Ansprüche 1 bis 4, **dadurch gekennzeichnet, daß** die Chromatographie an einem Anionenaustauscher bei einer lonenkonzentration von 0 bis 50 mMol NaCI und einem pH-Wert von 6,0 bis 8,0 durchgeführt wird.

8. Verfahren nach einem der Ansprüche 1, 3 oder 5 **dadurch gekennzeichnet, daß** die Chromatographie an einem Kationenaustauscher bei einer lonenkonzentration von 50 bis 200 mMol NaCI und einem pH-Wert von 4,0 bis 6,0 durchgeführt wird.

9. Verfahren gemäß einem der Ansprüche 1 bis 8, **dadurch gekennzeichnet, daß** vor der chromatographischen Reinigung gemäß Schritt b) eine Sterilisation durch Behandlung mit β-Propiolacton, TNBP/Tween, TNBP/Na-cholat oder TNBP erfolgt.

10. Verfahren gemäß Anspruch 9, **dadurch gekennzeichnet, daß** die Sterilisation zusätzlich in Kombination mit UV-Bestrahlung vorgenommen wird.

11. Verfahren gemäß Anspruch 10, **dadurch gekennzeichnet, daß** die Sterilisation durch Behandlung mit 0,03-0,1 Vol.-% β-Propiolacton und UV-Bestrahlung erfolgt.

12. Verfahren nach einem der Ansprüche 1 bis 11, **dadurch gekennzeichnet, daß** als Ausgangsmaterial eine Immunglobulin-G-haltige Fraktion mit hohen Antikörpertitern gegen virale, bakterielle oder zelluläre Antigene verwendet wird.

## Claims

1. Process for the production of an intravenously tolerable polyclonal immunoglobulin G solution from an immunoglobulin-G-containing starting material freed from coagulation factors selected from a gamma globulin fraction obtained by Cohn fractionation (Cohn paste II or II/III) or a crude immunoglobulin-G-containing solution chromatographically prepurified from serum **characterized in that**
a) the starting material, worked up in the usual way, is treated with 0.4 to 1.5 vol% octanoic acid, the solution is subsequently purified and
b) the solution so obtained is chromatographed on anion or cation exchangers or via a hydrophobic interaction chromatography.

2. Process according to Claim 1, **characterized in that** the immunoglobulin-G-containing solution is treated in step a) with 0.8 to 1 vol% octanoic acid.

3. Process according to one of Claims 1 or 2, **characterized in that** the chromatography is carried out on phases suitable for high-performance liquid chromatography (HPLC).

4. Process according to one of Claims 1 to 3, **characterized in that** QMA-Accell® or DEAE-Spherosil® is used as anion exchanger.

5. Process according to one of Claims 1 to 3, **characterized in that** CM-Accell®, SP-Spherodex®, SP-Trisacryl-LS®, or Fractogel TSK SP 650® is used as cation exchanger.

6. Process according to one of Claims 1 to 3, **characterized in that** the chromatography is carried out on Polypropyl A® as hydrophobic phase.

7. Process according to one of Claims 1 to 4, **characterized in that** the chromatography is carried out on an anion exchanger at an ionic concentration of 0 to 50 mmolar NaCI and a pH value of 6.0 to 8.0.

8. Process according to one of Claims 1, 3 or 5, **characterized in that** the chromatography is carried out on a cation exchanger at an ionic concentration of 50 to 200 mmolar NaCI and a pH value of 4.0 to 6.0.

9. Process according to one of Claims 1 to 8, **characterized in that** before the chromatographic purification according to step b) a sterilization is carried out by treatment with β-propiolactone, TNBP/Tween, TNBP/sodium cholate or TNBP.

10. Process according to Claim 9, **characterized in that** the sterilization is carried out in combination with UV irradiation in addition.

11. Process according to Claim 10, **characterized in that** the sterilization is carried out by treatment with 0.03-0.1 vol% β-propiolactone and UV irradiation.

12. Process according to one of Claims 1 to 11, **characterized in that** an immunoglobulin-G-containing fraction with high antibody titres against viral, bacterial or cellular antigens is used as starting material.

## Revendications

1. Procédé de préparation d'une solution d'immunoglobuline G polyclonale, tolérée par voie intraveineuse, à partir d'une substance de départ contenant de l'immunoglobuline G, exempte de facteurs de coagulation choisi à une fraction de γ-globuline obtenue par fractionnement de Cohn (fraction de Cohn II ou II/III) ou une solution contenant de l'immunoglobuline G brute, pré-purifiée par chromatographie à partir de sérum **caractérisé en ce que**
a) on traite la substance de départ préparée de façon usuelle par 0,4 à 1,5 % en volume d'acide caprylique, qu'on purifie ensuite la solution et
b) on procède à la chromatographie de la solution ainsi obtenu sur des échangeurs d'anions ou de cations ou par chromatographie d'interaction hydrophobe.

2. Procédé selon la revendication 1, **caractérisé en ce que** la solution contenant de l'immunoglobuline G est traitée à l'étape a) par 0,8 à 1% en volume d'acide caprylique.

3. Procédé selon l'une des revendications 1 ou 2, **caractérisé en ce que** la chromatographie s'effectue sur des phases convenant à la chromatographie en phase liquide haute performance (HPLC).

4. Procédé selon la revendication 1 à 3, **caractérisé en ce qu'**on utilise comme échangeur d'anions QMA-Accell® ou DEAE-Spherosil®.

5. Procédé selon la revendication 1 à 3, **caractérisé en ce qu'**on utilise comme échangeur de cations CM-Accell®, SP-Spherodex®, SP-Trisacryl-LS® ou Fraktogel TSK SP 650®.

6. Procédé selon la revendication 1 à 3, **caractérisé en ce que** la chromatographie s'effectue sur Polypropyl-A® en tant que phase hydrophobe.

7. Procédé selon l'une des revendications 1 à 4, **caractérisé en ce que** la chromatographie s'effectue sur un échangeur d'anions pour une concentration ionique de 0 à 50 mmol de NaCI et à un pH de 6,0 à 8,0.

8. Procédé selon l'une des revendications 1, 3 ou 5, **caractérisé en ce que** la chromatographie s'effectue sur un échangeur de cations pour une concentration ionique de 50 à 200 mmol de NaCI et à un pH de 4,0 à 6.0.

9. Procédé selon l'une des revendications 1 à 8, **caractérisé en ce que** la purification par chromatographie selon l'étape b) est précédée par une stérilisation par traitement par la β-propiolactone, TNBP/Tween, TNBP/cholate de sodium ou TNBP.

10. Procédé selon la revendication 9, **caractérisé en ce qu'**on procède a la stérilisation additionnellement en association avec une irradiation UV.

11. Procédé selon la revendication 10, **caractérisé en ce que** la stérilisation s'effectue par traitement par 0,03 à 0,1% en volume de β-propiolactone et irradiation UV.

12. Procédé selon l'une des revendications 1 à 11, **caractérisé en ce qu'**on utilise comme substance de départ une fraction contenant de l'immunoglobuline G, ayant des titres élevés d'anticorps dirigés contre des antigènes viraux, bactériens ou cellulaires.
